# EUROPÄISCHE PATENTANMELDUNG

(11) **EP 2 465 557 A1**
(43) Veröffentlichungstag der Anmeldung: **20.06.2012**
(21) Anmeldenummer: 10195701.7
(22) Anmeldetag: 17.12.2010
(51) Int. Cl.: A61M 5/162

(54) **Vorrichtung für die Abgabe einer Flüssigkeit aus einem Behälter**

(71) Anmelder: Weibel CDS AG, 9104 Waldstatt (CH)
(72) Erfinder: Weibel, Ludwig Daniel, 9104 Waldstatt (CH)
(74) Vertreter: Hepp Wenger Ryffel AG

(57) **Zusammenfassung**

Die Erfindung betrifft eine Vorrichtung (2) für die Abgabe einer Flüssigkeit aus einem Behälter (3), welcher mit einem penetrierbaren Verschlusselement (14) verschlossen ist, umfassend einen rohrförmigen Hohlkörper (4) mit einem Innenraum (11), welcher an einem Längsende einen Abschluss (6) mit einer Abgabeöffnung (7) aufweist. In einem Innenraum (11), ist ein Kolben (12) mantelseitig fluiddicht und längsverschiebbar angeordnet, welcher im Innenraum (11) einen Vorratsbereich (31) und einen Abgabebereich (30) abgrenzt. Der Vorratsbereich (31) ist dabei nach aussen offen und kommuniziert mit der Atmosphäre. Dabei ist der Vorratsbereich (31) zur ganz oder teilweisen längsverschiebbaren Aufnahme des Behälters (3) ausgebildet. Der Abgabebereich (30) ist für eine Aufnahme einer zur Abgabe vorgesehenen Flüssigkeitsmenge vorgesehen und schliesst an die Abgabeöffnung (7) an. Der Kolben (12) umfasst Kopplungsmittel (27), mit welchem der Kolben (12) an ein entsprechendes Kopplungsmittel (33) des Behälters (3) bezüglich einer Längsverschiebung koppelbar ist. Der Kolben (12) weist weiter ein Penetrationselement (13) auf, mit welchem das Verschlusselement (14) penetrierbar ist, wenn der Kolben (12) an den Behälter (3) gekoppelt ist. Das Penetrationselement (13) weist einen Fluidkanal (24) auf, welcher einen Flüssigkeitsdurchtritt in den Abgabebereich (30) ermöglicht. Das Penetrationselement (13) umfasst einen Belüftungskanal (28) zum Belüften des Behälters (3) bei Entnahme der Flüssigkeit, welcher mit dem Vorratsbereich (31) kommuniziert und durch welchen dem Behälter (3) über den Vorratsbereich (31) Luft aus der Atmosphäre zuführbar ist.

## Beschreibung

Die Erfindung betrifft eine Vorrichtung für die Abgabe einer Flüssigkeit aus einem Behälter gemäss Oberbegriff des Anspruchs 1. Die Erfindung betrifft weiter einen zur Benutzung mit einer derartigen Vorrichtung geeigneten Behälter sowie eine Anordnung aus einer derartigen Vorrichtung und einem geeigneten Behälter. Im Weiteren betrifft die Erfindung auch Injektionsports für Dialysegeräte.

Es ist weitgehend bekannt, Spritzen zur Injektion von flüssigen Medikationen z.B. an Patienten, Behälter oder Katheter anzuwenden. Typischerweise sind die Spritzen dabei leer und ein Abgabevolumen im Spritzenkörper muss zunächst von einem Anwender aus einer Fiole oder einem Medikationsbehälter durch Erzeugung eines Unterdrucks infolge Ausziehen eines Kolbens mit der gewünschten Medikation befüllt werden. Dabei ist auch auf eine Wahrung der Sterilität zu achten, was bei getrennter Handhabung von Behälter und Spritze mitunter schwierig zu gewährleisten ist.

Um dieses Problem zu umgehen sind vorgefüllte Spritzen bekannt, bei welchen die Medikation bereits im Abgabevolumen der Spritze vorhanden ist. Diese haben allerdings gewisse Nachteile hinsichtlich der Lagerung der Spritzen, da hierzu beispielsweise ein Drücker, mit welchem der Kolben der Spritze verschoben werden kann, weitgehend vollständig ausgefahren sein muss. Die Lagerung der Spritze in befülltem Zustand ist somit vergleichsweise platzintensiv und es müssen Vorkehrungen getroffen werden, dass der weit überstehende Drücker nicht unbeabsichtigt betätigt wird. Ausserdem bestehen oft ungewünschte Wechselwirkungen zwischen den Materialen der Spritze und der Medikation, womit die Lagerfähigkeit beschränkt sein kann bzw. gar nicht möglich ist (z.B. Degeneration einer Dichtung am Kolben, Zerfall der Medikation infolge Kontakts mit gewissen Kunststoffen etc.). Die grösste unerwünschte Wechselwirkung besteht dabei mit einer Silikonbeschichtung, welche erforderlich ist, damit sich der Kolben auch nach längerer Zeit im Spritzenkörper problemlos bewegen lässt.

Der Stand der Technik schlägt hierzu vor, den Drücker selbst als Medikationsbehälter z.B. als eine Fiole auszubilden. Erst kurz vor der Benutzung wird die Fiole geöffnet, beispielsweise perforiert, wodurch die Medikation durch Ausziehen des Drückers in das Abgabevolumen der Spritze gebracht wird. Zur Abgabe der Medikation kann der Drücker analog herkömmlicher Spritzen wieder in den Spritzenkörper eingeschoben werden, wodurch über den Kolben das Abgabevolumen verkleinert und so die Medikation durch eine Abgabeöffnung abgegeben wird. Dabei kann auch eine weitere Medikation, beispielsweise in fester Form als Pulver, getrennt von der ersten Medikation im Behälter im Abgabevolumen der Spritze vorhanden sein. Erst wenn die erste Medikation in das Abgabevolumen eingebracht wird, erfolgt eine Durchmischung der beiden Medikationen, was eine Lagerung von gemeinsam zu verabreichenden, aber nicht in Mischung lagerfähigen Medikationen ermöglicht.

Eine derartige Vorrichtung ist in der WO 01/00261 (Baxter International Inc.) beschrieben. Diese Vorrichtung umfasst am Kolben ein gegen einen als Medikations-Behälter ausgebildeten Drücker gerichtetes Penetrationselement. Dem Penetrationselement zugewandt weist der Medikations-Behälter ein penetrierbares Verschlusselement auf. Durch Eindrücken des Drückers in den Spritzenkörper durchdringt das Penetrationselement das Verschlusselement wo es über widerhakenartige Vorsprünge im Verschlusselement verhakt. Beim Aufziehen der Spritze, d.h. beim Ausziehen des Drückers aus dem Spritzenkörper, wird der mit dem Drücker verhakte Kolben mitgenommen. Bei verschlossener Abgabeöffnung entsteht somit ein Unterdruck im Abgabevolumen, wodurch die Medikation durch einen Fluidkanal des Penetrationselements aus dem Behälter in den Abgabebereich gesogen wird. Beim Eindrücken des Drückers in den Spritzenkörper kann in der Folge die Medikation aus dem Abgabevolumen über die Abgabeöffnung abgegeben werden. Beim Ansaugen der Flüssigkeit entsteht allerdings ein Unterdruck im Behälter, wodurch ein Grossteil der Flüssigkeit wieder in den Behälter zurückströmt, wenn das Aufziehen unterbrochen wird. Zum Druckausgleich ist daher an einem aussen liegenden Betätigungsende des Drückers eine Belüftungsöffnung in Form eines Ventils zur Zuführung von Luft aus der Atmosphäre in den Behälter vorgesehen.

Während die Zuführung von Luft grundsätzlich eine vorteilhafte Massnahme bei der Entnahme der Medikation aus dem Behälter darstellt, besteht im oben genannten Fall das Risiko einer mikrobiologischen Verunreinigung der Medikation durch Ansaugen verunreinigter Luft. Insbesondere am Betätigungsende des Drückers ist allgemein mit einer erhöhten Kontamination zu rechnen. Ebenso besteht die Gefahr eine Obstruktion der Belüftungsöffnung durch den Anwender bei der Handhabung der Spritze.

Es ist daher die Aufgabe der Erfindung, eine Vorrichtung der eingangs genannten Art zu schaffen, welche die Nachteile des Standes der Technik überwindet und bei der insbesondere unter Aufrechterhaltung von sterilen Bedingungen ein Rückströmen von Flüssigkeit in den Behälter vermieden wird. Die Vorrichtung soll ausserdem zuverlässig funktionieren und auch von nicht besonders geschultem Personal sicher angewendet werden können. Ausserdem soll die Vorrichtung einfach und kostengünstig hergestellt werden können, da die Vorrichtung auch nur zum einmaligen Gebaruch vorgesehen sein kann.

Diese Aufgaben werden erfindungsgemäss mit einer Vorrichtung gelöst, welche für die Abgabe einer Flüssigkeit aus einem Behälter, welcher mit einem penetrierbaren Verschlusselement verschlossen ist vorgesehen ist. Die Vorrichtung umfasst einen rohrförmigen Hohlkörper mit einem Innenraum, welcher an einem Längsende einen Abschluss mit einer Abgabeöffnung aufweist. Der Hohlkörper weist einen Innenraum auf, in welchem ein mantelseitig fluiddicht längsverschiebbarer Kolben angeordnet ist. Der Kolben grenzt im Innenraum einen Vorratsbereich und einen Abgabebereich ab, wobei der Vorratsbereich nach aussen offen ist und mit der Atmosphäre kommuniziert und zur ganz oder teilweisen längsverschiebbaren Aufnahme des Behälters ausgebildet ist. Der Abgabebereich ist für eine Aufnahme einer zur Abgabe vorgesehenen Flüssigkeitsmenge vorgesehen und schliesst an die Abgabeöffnung an. Der Kolben umfasst weiter ein Kopplungsmittel, mit welchem der Kolben an ein entsprechendes Kopplungsmittel des Behälters bezüglich einer Längsverschiebung koppelbar ist. Weiter weist der Kolben ein Penetrationselement auf, mit welchem das Verschlusselement penetrierbar ist, wenn der Kolben an den Behälter gekoppelt ist. Das Penetrationselement weist dabei einen Fluidkanal auf, welcher einen Flüssigkeitsdurchtritt in den Abgabebereich ermöglicht. Die Vorrichtung zeichnet sich dadurch aus, dass das Penetrationselement einen Belüftungskanal zum Belüften des Behälters bei Entnahme der Flüssigkeit umfasst, welcher mit dem Vorratsbereich kommuniziert und durch welchen dem Behälter über den Vorratsbereich Luft aus der Atmosphäre zuführbar ist.

Im Folgenden wird eine Richtung zum Längsende des Hohlkörpers, welches mit der Abgabeöffnung versehen ist, mit einer "Richtung nach vorne" oder einfach "vorne" bezeichnet, während eine Richtung zu einem gegenüberliegenden Längsende mit einer "Richtung nach hinten" oder kurz "hinten" bezeichnet wird.

Der wenigstens eine, vorzugsweise entlang des bevorzugt als Hohlnadel ausgebildeten Penetrationselements verlaufende, Belüftungskanal bewirkt, dass das Innere des Behälters mit der Atmosphäre kurzgeschlossen ist, wenn das Penetrationselement durch das Verschlusselement in den Innenraum des Behälters ragt. Der Aufbau eines stetig steigenden Unterdrucks im Behälter wird damit vermieden, da jeweils so viel Luft aus der Atmosphäre in den Behälter nachströmt, wie Flüssigkeit entfernt wird. Erfindungsgemäss wird dem Belüftungskanal durch den Vorratsbereich der Vorrichtung Luft aus der Atmosphäre zugeführt. Eine Einlassöffnung ist somit im Innenraum des Hohlkörpers angeordnet, womit sie vor einem direkten Zugriff durch einen Anwender geschützt ist. Durch die Anordnung der Einlassöffnung besteht somit eine erste Barriere gegen eine Kontamination der in den Belüftungskanal eintretenden Luft mit Schmutz oder Keimen.

Selbstverständlich ist es denkbar, mehrere Lüftungskanäle anzuordnen, welche entlang des Penetrationselements entweder linear oder auch schraubenlinienförmig verlaufen können. Denkbar wäre auch die Anordnung eines Belüftungskanals an einem von dem Penetrationselement getrennten Element, beispielsweise in der Form einer separaten Belüftungsnadel.

Der Innenraum des Hohlkörpers kann dabei abschnittweise einen unterschiedlichen Querschnitt und/oder Durchmesser aufweisen. Dies ist insbesondere dann von Vorteil, wenn die Vorrichtung für die Abgaben kleinster Mengen der Flüssigkeit vorgesehen ist. Der Abgabebereich ist in diesem Fall mit Vorteil in einem Bereich mit kleinerem Querschnitt angeordnet, als ein Querschnitt im Vorratsbereich. Damit kann eine im Abgabebereich vorhandene Flüssigkeitsmenge z.B. mittels einer aufgedruckten Skala genauer abgemessen werden, während ausreichend Platz für den Behälter sowie die Kopplungsmittel und das Penetrationselement im Vorratsbereich bereitgestellt ist. In diesem Fall erstreckt sich der Kolben vorzugsweise von dem Bereich mit grösserem Querschnitt derart in den Bereich mit kleinerem Querschnitt, dass der Kolben dennoch möglichst über die gesamte Länge des Abgabebereichs verschiebbar ist (z.B. zwei Kolbenplatten unterschiedlichen Durchmessers, welche über eine Druckstange entsprechender Länge fest miteinander verbunden sind). Damit kann ein konstruktiv aufwändigerer Kolbenteil mit dem Kopplungsmittel und Penetrationselement im Bereich des grösseren Querschnitts angeordnet sein, während ein im Bereich mit kleinerem Querschnitt angeordneter Kolbenteil nur für das Ausstossen der Flüssigkeit aus dem Abgabebereich ausgebildet zu sein braucht (und damit konstruktiv weniger aufwändig ist).

In einer bevorzugten Ausführungsform ist ein Filter vorgesehen, über das Luft aus der Atmosphäre über den Belüftungskanal in den Behälter zuführbar ist. Das Filter ist dabei derart an der Vorrichtung angeordnet, dass die durch den Belüftungskanal zugeführte Luft das Filter passieren muss. Vorzugsweise ist das Filter dem Belüftungskanal atmosphärenseitig vorgelagert. Das Filter kann dabei am Kolben angeordnet sein, welcher in diesem Fall bevorzugt auch die Einlassöffnung des Belüftungskanals umfasst. Damit wird zusätzlich sichergestellt dass die dem Behälter zugeführte Luft eine schmutzfreie und sogar keimfreie Umgebung im Behälter gewährleistet. Je nach Beschaffenheit dieses Filters können auch kleinste Partikel oder Lebewesen zurückgehalten werden.

Das Filter kann allerdings auch am Behälter vorgesehen sein, sodass die in den Belüftungskanal eintretende Luft den Filter erst passieren muss, wenn der Behälter in der Vorrichtung angeordnet und der Kolben mit dem Behälter gekoppelt ist (siehe unten).

Werden geringere Ansprüche an die Sterilität gestellt, z.B. bei einer nicht-invasiven Medikation wie z.B. im Kosmetikbereich (z.B. gezieltes Auftragen von Pasten o.ä.), kann zur Kostenersparnis auch gänzlich auf das Filter verzichtet werden.

Gemäss einer bevorzugten Ausführungsform umfasst das Penetrationselement eine Hohlnadel, deren Hohlraum zumindest einen Teil des Fluidkanals bildet. In diesem Fall ist der Belüftungskanal besonders vorteilhaft als Nut auf der Aussenseite der Hohlnadel ausgebildet. Vorzugsweise sind es mehrere parallele Nuten. Ein derartiges Bauteil ist einfach herzustellen und bei ausreichender Nuttiefe wird selbst dann ein genügend grosser Durchtrittsquerschnitt freigehalten, wenn das Verschlusselement aus sehr weichem und elastischem Material besteht. Es versteht sich, dass auch bei beliebigen Ausbildungen des Penetrationselements der Belüftungskanal mit Vorteil als aussenseitige Nut ausgebildet ist, wobei auch in diesem Fall vorzugsweise mehrere Nuten vorhanden sind.

In einer bevorzugten Ausführungsform ist ein Ventil ausgebildet welches einen Durchtritt von Flüssigkeit durch den Fluidkanal in den Abgabebereich zulässt und in Gegenrichtung sperrt. Besonders vorteilhaft ist ein so genanntes Domventil, eine kuppelförmige zumindest teilweise elastische Membran mit einem Schlitz für einen Flüssigkeitsdurchtritt. Das Domventil ist bevorzugt mit der Kuppelwölbung zum Abgabebereich hin mit Vorteil im Fluidkanal oder an einer Öffnung des Fluidkanals angeordnet. In einer besonders bevorzugten Ausführung ist das Ventil an den Kolben angeformt, insbesondere einstückig mit dem Kolben ausgebildet. In Varianten können auch andere geeignete Arten von Ventilen wie z.B. so genannte Schnabelventile ("duckbill") oder Schirmventile zum Einsatz kommen. In einer bevorzugten Ausführungsform ist eine Ventil Schliess- oder Rückstellkraft dabei derart bemessen, dass nach einer Punktion einer Vene beim Zurückziehen des Kolbens Blut in den Abgabebereich strömt, ohne dass Flüssigkeit oder Luft aus dem Behälter in den Abgabebereich gelangt. Durch das Aufziehen einer geringen Menge Blut kann somit analog zu bekannten herkömmlichen Spritzen kontrolliert werden, ob die Vene getroffen wurde. Die Schliesskraft des Ventils bezüglich eines Flüssigkeitsflusses aus dem Behälter in den Abgabebereich muss also genügend gross sein, um den vom Fluidkanal des Penetrationselements gebildeten Zugang zum Behälter verschlossen zu halten, wenn ein durch das Zurückziehen des Kolbens erzeugter Unterdruck im Abgabebereich genügend gross ist, um Blut durch die Abgabeöffnung aus der Vene anzusaugen. Die gewünschte Schliesskraft des Ventils kann hierfür beispielsweise durch geeignete Materialwahl und/oder durch gezielte Dimensionierung der Materialstärke wie z.B. durch eine gezielte Ausdünnung erreicht werden.

Mit Vorteil ist auch die Abgabeöffnung mit einem Ventil versehen, welches einen Austritt von Flüssigkeit durch die Abgabeöffnung aus dem Abgabebereich zulässt und in Gegenrichtung sperrt. Das Ventil verhindert so eine Kontamination einer im Abgabevolumen vorhandenen Medikation. Je nach Erfordernis können dabei unterschiedliche Ventilsysteme zur Anwendung kommen. Neben den vorgenannten Ventilarten sind in diesem Zusammenhang insbesondere auch Schirmventile zu erwähnen, welche im Zusammenhang mit an die Vorrichtung angeformten Anschlussstücken zur Ankopplung an ein fluidführendes System besonders vorteilhaft sein können (siehe unten).

In einer bevorzugten Ausführungsform ist das Kopplungsmittel, mit welchem der Kolben an ein entsprechendes Kopplungsmittel des Behälters bezüglich einer Längsverschiebung koppelbar ist, als Teil einer Schnapp-Kupplung ausgebildet. Schnapp-Kupplung oder Klick-Kupplung bezeichnet hierbei ein Kopplungsmittel, welches bei axialem Eingriff durch Verrastung automatisch eine formschlüssige Verbindung herstellt (Rastnasen/ Rastkerben, Haken/Ösen etc.). In Varianten kann je nach Erfordernis auch eine Dreh-Kupplung wie z.B. ein Gewinde oder ein Bajonett zur Anwendung kommen. In diesem Fall ist zum Kopplungs-Schluss allerdings neben einem axialen Eingriff auch eine Rotationsbewegung erforderlich, was unter anderem auch eine zusätzliche Verdrehsicherung des Kolbens voraussetzt.

In einer weiteren bevorzugten Ausführungsform weist der Hohlkörper an einem dem Abschluss gegenüberliegenden Längsende eine Zugangsöffnung auf, welche einen Zugang zum Vorratsbereich bildet und derart ausgestaltet ist, dass der Behälter durch die Zugangsöffnung in den Vorratsbereich einbringbar und/oder zum Verschieben im Innenraum von aussen manipulierbar ist. In einer besonders einfachen Konstruktion ist der Holkörper dabei weitgehend über den gesamten Querschnitt nach hinten offen. Die Zugangsöffnung kann dabei ähnlich wie bei bekannten Spritzen ausgebildet sein, sodass ein Medikationsbehälter, der in dem Vorratsbereich angeordnet ist, nach hinten über den Hohlkörper übersteht und als Drücker z.B. zum Verschieben des Kolbens benutzt werden kann. Die Zugangsöffnung bildet bevorzugt gleichzeitig eine Ansaugöffnung für die zur Zuführung in den Behälter vorgesehene Luft aus der Atmosphäre.

Mit Vorteil weist die Vorrichtung ein Sicherungsmittel auf, insbesondere in einem Bereich an der Zugangsöffnung, welches ein unbeabsichtigtes Ausbringen des Behälters aus dem Innenraum verhindert, wenn der Behälter, zumindest teilweise, im Innenraum angeordnet ist. Vorzugsweise umfasst das Sicherungsmittel einen elastischen Ring, beispielsweise einen Silikonring. Der Ring ist dabei derart bemessen und im Vorratsvolumen bzw. an der Zugangsöffnung angeordnet, dass der Behälter problemlos von einem Anwender durch den Ring in das Vorratsvolumen geschoben werden kann, aber dennoch durch Kraftschluss vom Ring an seiner Aussenwand gegen ein Herausfallen gesichert ist. Mit Vorteil umgreift der Ring eine entsprechend ausgebildete aussenseitige Verjüngung des Behälters (Hinterschnitt), wenn dieser in einer gewünschten, beispielsweise für die Lagerung vorgesehenen, Position im Vorratsbereich angeordnet ist.

Der Ring weist dabei bevorzugt Massnahmen auf, welche auch bei im Vorratsbereich angeordnetem Behälter einen ungehinderten Durchtritt von Luft aus der Atmosphäre in den Vorratsbereich erlauben (z.B. Durchbrüche oder eine nach innen gerichtete Zahnung). In einer gegebenenfalls zu bevorzugenden Variante kann das Sicherungsmittel auch an einer Verschlusskappe vorgesehen sein, welche einen Abschluss zur sterilen Versiegelung des Innenraums der Zugangsöffnung des Hohlkörpers bildet und bei Gebrauch wenigstens teilweise abgenommen wird.

Zur Befestigung eines derartigen Abschlusses zur sterilen Versiegelung des Innenraums, z.B. zur Lagerung, weist der Hohlkörper vorzugsweise im Bereich an der Zugangsöffnung entsprechende Mittel zur Befestigung auf.

Mit Vorteil umfasst das Mittel zur Befestigung des Abschlusses einen umlaufenden, nach aussen auskragenden Flansch, an welchem ein z.B. als Verschlusskappe ausgebildeter Abschluss dichtend aufsetzbar und befestigbar ist. Die Verschlusskappe kann dabei bevorzugt am Flansch z.B. ultraschall- oder laserverschweisst werden, sodass die Kappe von einem Anwender mit verhältnismässigem Aufwand durch Bruch der Verbindung entfernt werden kann. Die Verschlusskappe kann aber auch eine Sollbruchstelle umfassen und fest mit dem Hohlkörper verbunden sein, sodass die Kappe längs der Sollbruchstelle entfernt wird, während ein Rest als Garantieband am Hohlkörper verbleibt.

Der Flansch kann gleichzeitig als Fingerrast ausgebildet sein, um beim Betätigen der Spritze, d.h. beim Eindrücken des Drückers bzw. des Behälters, eine entsprechende Gegenkraft auf den Hohlkörper auszuüben. In einer gegebenenfalls zu bevorzugenden Variante sind keine separaten Mittel zur Befestigung vorgesehen und ein hinterer Bereich des Hohlkörpers selbst ist als Abschluss ausgebildet. Dabei sind mit Vorteil Sollbruchstellen vorgesehen, sodass der hintere Bereich geöffnet, z.B. auseinandergeklappt, werden kann und somit die Zugangsöffnung freigibt. Der hintere Bereich des Hohlkörpers kann in diesem Fall gleichzeitig die sterile Versiegelung (eingeklappt, mit Sollbruchstelle - z.B. Schweissnaht) und eine Fingerrast (ausgeklappt) bilden.

Während in verschiedenen Anwendungsbereichen der Vorrichtung eine blosse Abgabe der Flüssigkeit an der Abgabeöffnung ausreicht ist es insbesondere im medizinischen Bereich von Vorteil, wenn der Hohlkörper aussenseitig an der Abgabeöffnung ein Kupplungselement aufweist. Mittels des Kupplungselements kann die Vorrichtung z.B. direkt an fluidführende Systeme angekoppelt werden oder es können Injektions- oder Spülnadeln aufgesetzt werden. Mit Vorteil wird dabei auf bekannte und normierte Kupplungssysteme zurückgegriffen. Insbesondere umfasst das Kupplungselement daher einen Aussenkonus eines Luer-Locks, womit die Vorrichtung an entsprechende Normelemente fluidführender Systeme, insbesondere im medizinischen Bereich, angeschlossen werden kann. Es versteht sich, dass die Vorrichtung auch direkt mit beispielsweise einer eingeklebten oder eingesteckten Spülnadel oder Injektionsnadel versehen sein kann.

Beispielsweise ist es, insbesondere im Bereich von Dialysesystemen bekannt, Anschlussstücke mit Kopplungsstellen, so genannten Injektionsports, für medizinische Einrichtungen zur Abgabe einer Medikation in ein fluidführendes System einzusetzen. Derartige Anschlussstücke umfassen in der Regel einen Rohrabschnitt mit einem zur Fluidführung vorgesehenen Lumen, welches mit dem fluidführenden System kommuniziert. Zur Einbringung einer Medikation in das Fluidum kann beispielsweise eine Spritze an die Kopplungsstelle angeschlossen werden. Die Kopplungsstelle umfasst in diesem Fall eine Zugangsöffnung, durch welche die Medikation in das Lumen und somit in das Fluidum eingebracht werden kann. In der Regel ist dabei ein Ventil zum Abschluss der Zugangsöffnung vorhanden. Aussenseitig am Anschlussstück an der Kopplungsstelle können Kopplungselemente wie beispielsweise ein Luer-Lock vorgesehen sein, sodass entsprechend ausgebildete medizinische Einrichtungen fluiddicht und lösbar an die Zugangsöffnung angekoppelt werden können.

Derartige bekannte Anschlussstücke weisen bei den Zugangsöffnungen im Lumen jedoch häufig ein unerwünschtes Totraumvolumen auf. Gerade bei kleinsten eingebrachten Medikationsdosen ist somit nicht sichergestellt, dass die Medikation sich schnell und vollständig im Fluid verteilt. Zudem ist es oft erforderlich, miteinander inkompatible Medikationen in kurzer Folge zu verabreichen, weshalb eine längere Verweilzeit der zuerst verabreichten Medikation im Totraumvolumen zu einer unerwünschten Vermischung führen kann. Nicht zuletzt bergen Totraumvolumina auch das Risiko von Luftansammlungen, welche einen an das System angeschlossenen Patienten gefährden können.

Es ist daher eine weitere Aufgabe der Erfindung, die Nachteile bekannter Anschlussstücke zu überwinden und ein Anschlussstück bereitzustellen, welches keine oder nur geringe Totraum-Volumina aufweist und vielseitig anwendbar ist. Ausserdem soll das Anschlussstück auch kostengünstig in der Herstellung sowie sicher und einfach in der Anwendung sein.

Eine besonders vorteilhafte Ausführung eines Anschlussstücks umfasst hierzu ein rohrförmiges Gehäuse wie z.B. einen Rohrabschnitt mit einem Lumen zur Führung eines Fluidums. Das Gehäuse weist wenigstens eine Zugangsöffnung auf, welche in der Gehäusewand ausgebildet ist. Aussenseitig an der Zugangsöffnung ist ein Kopplungsmittel zur fluiddichten Ankopplung einer medizinischen Einrichtung zur Abgabe einer Medikation derart ausgebildet, dass die Medikation von der medizinischen Einrichtung durch die Zugangsöffnung in das Lumen einbringbar ist. Weiter ist eine Ventilvorrichtung vorhanden, welche einen Durchtritt von Flüssigkeit durch die Zugangsöffnung in das Lumen zulässt und in Gegenrichtung sperrt. Das Anschlussstück zeichnet sich dadurch aus, dass die Ventilvorrichtung ein Schirmventil umfasst, welches direkt an der Gehäusewand angebracht ist.

Hier und im Folgenden wird, sofern nicht anders vermerkt, eine Zugangsöffnung mit einem Kopplungsmittel zur fluiddichten Ankopplung einer medizinischen Einrichtung zur Abgabe einer Medikation allgemein als Injektionsport bezeichnet. Ein derartiger Injektionsport kann zusätzlich mit einem hygienisch unbedenklichen und einfach zu reinigenden Anschluss für die medizinische Einrichtung versehen sein, wie beispielsweise einem so genannten "abtupfbaren Ventil" ("swabable valve") gemäss der US 6,651,956 (Halkey-Roberts Corporation).

Bevorzugt liegt ein Schirm des Schirmventils direkt an einer Innenseite der Gehäusewand an, welche das Lumen begrenzt, und verschliesst so die in der Gehäusewand ausgebildete Zugangsöffnung. Der Schirm des Schirmventils ist bevorzugt als flache flanschartige, sich radial nach aussen verjüngende, elastische Dichtlippe ausgebildet. Der Schirm ist bevorzugt in einem zentralen Bereich des Schirms am Gehäuse befestigt. Der flache Schirm kann sich somit dichtend an die Innenwand des Gehäuses schmiegen und verringert einen Querschnitt des Lumens nur geringfügig. Insbesondere weist ein Übergang von der Innenwand auf den Schirm aufgrund der sich verjüngenden Form einen weitgehend glatten und stufenlosen Übergang auf, welcher unerwünschte Verwirbelungen im strömenden Fluidum weitgehend vermeidet.

Der Ventilmechanismus kann somit direkt im Lumen angeordnet sein. Insbesondere muss er nicht wie bei bekannten Systemen nach aussen zurückversetzt in einer Tasche oder einer Ausbuchtung am Gehäuse vorgesehen sein, um einen weitgehend konstanten Durchfluss-Querschnitt im Lumen zu ermöglichen. Das Gehäuse des Anschlussstücks kann daher in einer bevorzugten Ausführungsform eine weitgehend prismatische Form mit weitgehend konstantem Innenquerschnitt aufweisen.

Es versteht sich, dass an einer Kopplungsstelle auch mehrere Öffnungen vorhanden sein können, welche von demselben Schirmventil verschlossen sind. Bevorzugt sind im Fall mehreren Öffnungen diese symmetrisch um den zentralen Bereich des Schirms des Schirmventils angeordnet.

Endseitig kann das rohrförmige Gehäuse Kupplungselemente aufweisen, mit welchen das Anschlussstück an einem oder beiden Enden an das fluidführende System angeschlossen werden kann. In Varianten kann das Anschlussstück einseitig abgeschlossen sein oder direkt als Abschnitt eines Schlauchs oder Rohrs ausgebildet sein.

Mit Vorteil weist das Anschlussstück wenigstens eine weitere Zugangsöffnung mit Kopplungsmittel zum Anschluss einer medizinischen Einrichtung zur Abgabe einer Medikation auf. Bevorzugt sind auch ein oder mehrere penetrierbare und selbst dichtende Septa ausgebildet, sodass eine Medikation auch mit einer Injektionsnadel in das Lumen eingebracht werden kann. Die Septa überdecken dabei bevorzugt eine Öffnung in der Gehäusewand und sind beispielsweise durch ein Ultraschallverfahren mit dem Gehäuse verschweisst. Der Querschnitt des Lumens wird somit nicht oder nur geringfügig verändert, womit das Fluidum weitgehend unbeeinflusst strömen kann.

In einer bevorzugten Ausführungsform der oben beschriebenen erfindungsgemässen Vorrichtung für die Abgabe einer Flüssigkeit aus einem Behälter ist das Kupplungselement an der Abgabeöffnung direkt als Anschlussstück wie oben beschrieben ausgebildet. Insbesondere heisst dies, dass das Kupplungselement der erfindungsgemässen Vorrichtung für die Abgabe einer Flüssigkeit aus einem Behälter einen weitgehend quer zur Längsachse des Holkörpers angeordneten Rohrabschnitt mit einem zur Fluidführung vorgesehenen Lumen umfasst, wobei die Abgabeöffnung der Vorrichtung derart mit dem Lumen kommuniziert, dass eine im Abgabebereich vorhandene Flüssigkeit durch die Abgabeöffnung in das Lumen eingebracht werden kann.

Bevorzugt ist der Rohrabschnitt einstückig an den Hohlkörper der Vorrichtung angeformt, insbesondere angespritzt. In Varianten kann er auch als separates Teil ausgebildet sein, wie beispielsweise als Rohrabschnitt oder Schlauch, welcher mit der Vorrichtung verklebt, ultraschall-verschweisst oder auf andere Weise kraft-, form- oder stoffschlüssig verbunden ist.

Analog zum oben beschriebenen separaten Anschlussstück weist der Rohrabschnitt der Vorrichtung wenigstens an einem seiner Längsenden ein Kopplungselement zum Anschluss an eine fluidführende Komponente auf. Die gesamte Vorrichtung mit daran angebrachtem Rohrabschnitt kann somit auf einfache Weise in das fluidführende System integriert werden.

Je nach Erfordernis ist der Rohrabschnitt mit Vorteil integraler Bestandteil eines zur Fluidführung vorgesehenen Systems, insbesondere eines Schlauchs. In diesem Fall ist der Rohrabschnitt als Abschnitt eines Schlauchs oder Rohrs ausgebildet, welcher direkt mit der erfindungsgemässen Vorrichtung verbunden ist. Dies hat den Vorteil, dass beim Aufbau eines fluidführenden Systems wie beispielsweise einer Dialyseanordnung nur ein Schlauch eingesetzt zu werden braucht, ohne dass weitere Verbindungen zu einem Anschlussstück hergestellt werden müssen oder dass eine separate Spritze angekoppelt werden muss.

In einer bevorzugten Ausführungsform weist der Rohrabschnitt in einer Gehäusewand des Rohrabschnitts wenigstens einen Injektionsport zum Einbringen einer Flüssigkeit in das Lumen auf. Wie oben beschrieben umfasst der Injektionsport eine Zugangsöffnung sowie einem Kopplungsmittel, insbesondere ein Luer-Lock, zur fluiddichten Ankopplung einer medizinischen Einrichtung zur Abgabe einer Medikation. Vorzugsweise ist dabei eine Ventilvorrichtung vorhanden, insbesondere ein Schirmventil, welche einen Durchtritt von Flüssigkeit durch die Zugangsöffnung in das Lumen zulässt und in Gegenrichtung sperrt.

Der wenigstens eine, zusätzliche Injektionsport ermöglicht den Anschluss einer weiteren medizinischen Einrichtung wie beispielsweise einer Spritze um weitere Medikationen einbringen zu können. Insbesondere kann der Rohrabschnitt analog zum oben beschriebenen separaten Anschlussstück zusätzlich auch ein oder mehrere penetrierbare und selbst dichtende Septa aufweisen, sodass eine Medikation auch mit einer Injektionsnadel in das Lumen eingebracht werden kann.

Die Vorrichtung gemäss der Erfindung ist zur Anwendung mit einem Behälter für eine Flüssigkeit, insbesondere eine Medikation vorgesehen. Die Erfindung erfasst aber auch einen Behälter für eine Flüssigkeit, welcher für die Vorrichtung geeignet ist. Der Behälter weist hierzu eine mit einem penetrierbaren Verschlusselement verschlossenen Behältermündung auf und umfasst am Verschlusselement einen Aufsatz mit einem Kopplungsmittel zum Ankoppeln an den Kolben der Vorrichtung. Der Aufsatz weist dabei in einem für die Penetration vorgesehenen Bereich des Verschlusselements einen Durchtritt zum Durchtritt eines Penetrationselements auf. Der Aufsatz des Behälters umfasst weiter einen Belüftungskanal, welcher am Durchtritt offen ist, sodass einem im Durchtritt angeordneten Penetrationselement durch den Belüftungskanal Luft aus der Atmosphäre zuführbar ist.

Ist das Penetrationselement im Durchbruch angeordnet, schliesst dieses zur Verhinderung einer Kontamination mit Keimen oder Schmutz bevorzugt den Durchbruch derart fluid- und luftdicht ab, dass dem Penetrationselement nur noch über den Belüftungskanal des Aufsatzes Luft zugeführt werden kann. Mit Vorteil weist der Belüftungskanal im Aufsatz daher direkt hinter dem Durchtritt, d.h. zwischen Aufsatz und Verschlusselement, eine Auslassöffnung auf, sodass die zugeführte Luft einem beispielsweise als Nut an einer Hohlnadel ausgebildeten Belüftungskanal des Penetrationselements zuführbar ist. Bevorzugt ist der Belüftungskanal als Zwischenraum zwischen dem Aufsatz und dem Verschlusselement ausgebildet, insbesondere als entsprechende innenseitige Aussparung am Aufsatz.

Bevorzugt ist der Belüftungskanal hierzu an einer Stirnseite des Aufsatzes zur Atmosphäre hin offen. Die entsprechende Öffnung und/oder ein entsprechender Bereich des Kolbens, welcher vor der

Öffnung angeordnet ist, sind dabei derart ausgebildet, dass auch bei an den Behälter gekoppelten Kolben der Vorrichtung eine Zufuhr von Luft aus dem Vorratsbereich sichergestellt ist.

Bevorzugt umfasst in diesem Fall der Aufsatz des Behälters ein Filter, durch welches über den Belüftungskanal zu dem Penetrationselement, welches im Durchtritt angeordnet ist, Luft aus der Atmosphäre zuführbar ist. Insbesondere ist das Filter mit Vorteil im Bereich einer atmosphärenseitigen Öffnung des Belüftungskanals ausgebildet, sodass ein Eintritt von Keimen oder einer Verschmutzung in den Belüftungskanal von vorne herein verhindert wird. Indem das Filter am Behälter ausgebildet ist, kann die Vorrichtung grundsätzlich auch mehrmals, mit verschiedenen Behältern benutzt werden, wobei mit jedem Behälter automatisch ein frisches Filter bereitgestellt ist.

Besonders bevorzugt ist das Filter in einer Halterung am Aufsatz gehalten, welche den Durchtritt des Aufsatzes kreisringförmig umgibt. Somit ist sichergestellt, dass auch im Falle mehrerer Belüftungskanäle oder -öffnungen, welche um den Durchtritt herum verteilt angeordnet sein können, alle Öffnungen gleichmässig vom Filter überdeckt sind.

In einer bevorzugten Möglichkeit zur Befestigung des Aufsatzes auf einer Behältermündung ist der Aufsatz auf der Behältermündung aufgeschnappt. Bei entsprechend ausgebildeten Rastmitteln kann dabei eine relativ hohe Anpresskraft aufgebaut werden, welche eine gute Dichtigkeit gewährleistet. Vergleichbare Schnappverbindungen auf Behältermündungen sind beispielsweise bereits bei Verschlüssen in der Lebensmittelindustrie bekannt.

In einer Variante kann der Aufsatz auch mit einer umlaufenden Manschette erfolgen, welche um den Aufsatz und um die Behältermündung aufgeschrumpft, angerollt oder angebördelt ist. Bei einer Ausbildung dieser Manschette beispielsweise aus Aluminium oder aus einer Aluminiumlegierung kann das eine Ende der Manschette bei der Herstellung direkt in den Aufsatz eingegossen werden. Nach dem Füllen und Verschliessen des Behälters kann das freie Ende um die Behältermündung angerollt oder angebördelt werden.

Die Erfindung umfasst auch eine Anordnung aus einer erfindungsgemässen Vorrichtung sowie mit einem Behälter für eine Flüssigkeit, welcher eine mit einem penetrierbaren Verschlusselement verschlossene Behältermündung aufweist und am Verschlusselement einen Aufsatz mit einem Kopplungsmittel zum Ankoppeln an einen Kolben der Vorrichtung umfasst, insbesondere mit einem Behälter wie oben beschrieben, wobei der Behälter wenigstens teilweise im Vorratsbereich angeordnet ist. Der Behälter ist dabei bevorzugt derart angeordnet, dass er an einem hinteren Ende durch die Zugangsöffnung übersteht und so als Drücker benutzt werden kann. Bevorzugt umfasst die Anordnung in diesem Fall eine Abschlusskappe, welche die Zugangsöffnung vorzugsweise steril verschliesst.

Die Anordnung kann aber auch derart ausgebildet sein, dass der Behälter vollständig im Vorratsbereich angeordnet ist, und erst durch Öffnen wie z.B. Auseinanderklappen eines hinteren Bereichs des Hohlkörpers längs einer Sollbruchstelle, ein hinterer Bereich des Behälters freigegeben wird.

Die Anordnung ist bevorzugt in eine Aufbewahrungsstellung und eine Entnahmelage bringbar, wobei der Behälter in der Aufbewahrungsstellung derart im Vorratsbereich angeordnet ist, dass das Penetrationselement vom Verschlusselement des Behälters beabstandet ist. Gemäss dieser Anordnung kann durch Eindrücken des Behälters an einem hinteren Ende die Anordnung von der Aufbewahrungsstellung in eine Entnahmestellung gebracht werden, wobei das Penetrationselement das Verschlusselement durchdringt und bei geeignetem Kopplungsmittel der Behälter mit dem Aufsatz an den Kolben gekoppelt wird. Durch Ausziehen des Behälters wird der Kolben in der Folge mitgenommen, wodurch bei verschlossener Abgabeöffnung ein Unterdruck im Abgabebereich entsteht, welcher die Flüssigkeit durch den Fluidkanal aus dem Behälter in den Abgabebereich saugt. Über den Belüftungskanal des Penetrationselements wird ein dabei entstehender Unterdruck im Behälter ausgeglichen.

Dabei ist in der Entnahmelage mit Vorteil der Belüftungskanal des Penetrationselements derart bezüglich dem Belüftungskanal des Aufsatzes angeordnet, insbesondere derart im Durchtritt des Aufsatzes angeordnet, dass die Belüftungskanäle miteinander kommunizieren und somit ein weitgehend durchgehender Gesamt-Belüftungskanal gebildet ist, durch welchen dem Behälter über den Vorratsbereich Luft aus der Atmosphäre zuführbar ist.

Dabei sind vorzugsweise die Belüftungskanäle in der Entnahmelage derart zueinander angeordnet, dass die Luft aus der Atmosphäre bei der Zuführung zum Behälter von dem Vorratsbereich der Vorrichtung durch den Belüftungskanal des Aufsatzes in den Belüftungskanal des Penetrationselements eintritt.

Im Falle eines Filters am Aufsatz des Behälters ist das Filter mit Vorzug derart bezüglich der Belüftungskanäle angeordnet, dass die Luft aus der Atmosphäre zunächst durch den Filter tritt, bevor sie in den Belüftungskanal des Aufsatzes und/oder des Penetrationselements eintritt. Damit wird von vorne herein verhindert, dass die Belüftungskanäle mit Schmutz oder Keimen kontaminiert werden können.

Weitere Vorteile und Einzelmerkmale der Erfindung sind in den Zeichnungen dargestellt und werden nachstehend beschrieben. Es zeigen schematisch:
- Figur 1:: Einen Längsquerschnitt durch eine erfindungsgemässe Anordnung in einem vorderen Bereich bei der Abgabe-öffnung in der Lagerstellung;
- Figur 2:: die Anordnung gemäss Figur 1 in Entnahmelage;
- Figur 3:: ein der Figur 1 entsprechender Längsquerschnitt der Anordnung in der Lagerstellung in einem hinteren Bereich;
- Figur 4a-e:: den Ablauf einer Benutzung der Anordnung in 5 Schritten;
- Figur 5:: eine weitere Ausführungsform einer erfindungsgemässen Anordnung mit unterschiedlichen Innenquerschnitten des Hohlkörpers;
- Figur 6:: eine weitere Ausführungsform einer erfindungsgemässen Vorrichtung mit angeformtem Anschlussstück.

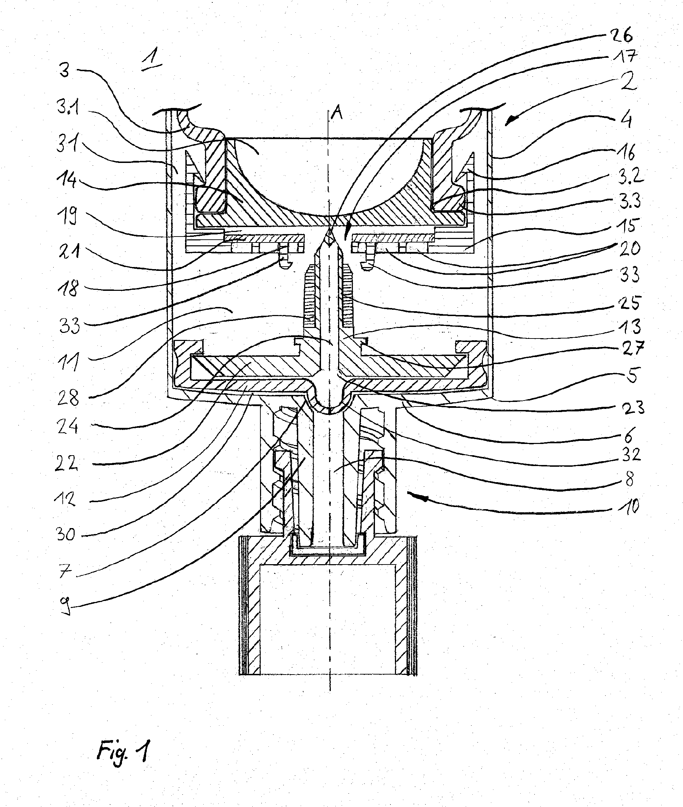

Figur 1 zeigt eine Anordnung 1 umfassend eine erfindungsgemässe Vorrichtung 2 sowie einen in der Vorrichtung 2 angeordneten Behälter 3. Die Vorrichtung 2 umfasst im Wesentlichen einen rohrförmigen Hohlkörper 4 mit einer Längsachse A. Der Hohlkörper 4 weist an einem vorderen Längsende 5 einen Abschluss 6 auf, in welchem eine zentrale, d.h. auf der Längsachse A angeordnete, Abgabeöffnung 7 ausgebildet ist. Die Abgabeöffnung 7 geht direkt in einen Fluidkanal 8 eines längs der Längsachse A aussenseitig am Abschluss angeordneten Aussenkonus 9 eines Luer-Locks 10 über und kommuniziert mit einem Innenraum 11 des Hohlkörpers 4.

Hinter der Abgabeöffnung 7 ist im Innenraum 11 des Hohlkörpers 4 ein Kolben 12 mit einem Penetrationselement 13 angeordnet, hinter welchem wiederum der Behälter 3 angeordnet ist. Der Behälter 3 ist länglich ausgebildet und weitgehend koaxial mit der Längsachse A angeordnet. Der Behälter 3 ist beispielsweise aus Glas gefertigt und mit einer Flüssigkeit 3.1 gefüllt. Eine Behältermündung 3.2 ist mit einem Verschlusselement 14, beispielsweise aus Gummi, luftdicht verschlossen und nach vorne dem Penetrationselement 13 des Kolbens 12 zugewandt. Bei der Flüssigkeit 3.1 kann es sich beispielsweise um einen pharmazeutischen Wirkstoff handeln, der dazu bestimmt ist, mit einer Injektionsspritze entweder direkt in den menschlichen oder tierischen Körper injiziert zu werden oder aber einem Infusionssystem beigemischt zu werden.

Der Behälter 3 umfasst einen Aufsatz 15 aus Kunststoffmaterial, der mit seiner Unterseite dichtend auf dem äusseren Randbereich des Verschlusselements 14 aufliegt, sodass das Verschlusselement 14 im Randbereich zwischen einem Rand der Behältermündung 3.2 und dem Aufsatz 15 eingeklemmt ist. Die dichtende und formschlüssige Verbindung des Aufsatzes 15 mit der Behältermündung 3.2 erfolgt über einen klauenartig ausgebildeten Kragen 16, welcher einstückig am Aufsatz 15 ausgebildet ist und nach hinten ragt. Der Kragen 16 übergreift dabei einen nach aussen auskragenden Wulst 3.3 an der Behältermündung 3.2 in der Funktion einer Schnapp-Kupplung. Der Aufsatz 15 kann somit mit dazwischen angeordnetem Verschlusselement 14 auf den Behälter 3 aufgeschnappt werden.

In einer Ebene unmittelbar vor dem Verschlusselement 14 weist der Aufsatz 15 einen zentralen Durchbruch 17 auf, welcher von einem Zentrierring 18 des Aufsatzes 15 kreisringförmig umgeben ist. Der Zentrierring 18 ist dabei derart am Aufsatz 15 ausgebildet und vom Verschlusselement 14 nach vorne beabstandet, dass sich zwischen Zentrierring 18 und Verschlusselement 14 ein zur Luftführung vorgesehener Belüftungskanal bzw. -raum 19 ergibt. Im Zentrierring 18 sind Ansaugöffnungen 20 ausgebildet, welche mit dem Belüftungskanal 19 kommunizieren und nach vorne offen sind. Am Zentrierring 18 ist auf der dem Verschlusselement 14 zugewandten Seite ein mikrobiologisch dichtes Filter 21 angeordnet, das sämtliche Ansaugöffnungen 20 vollständig überdeckt.

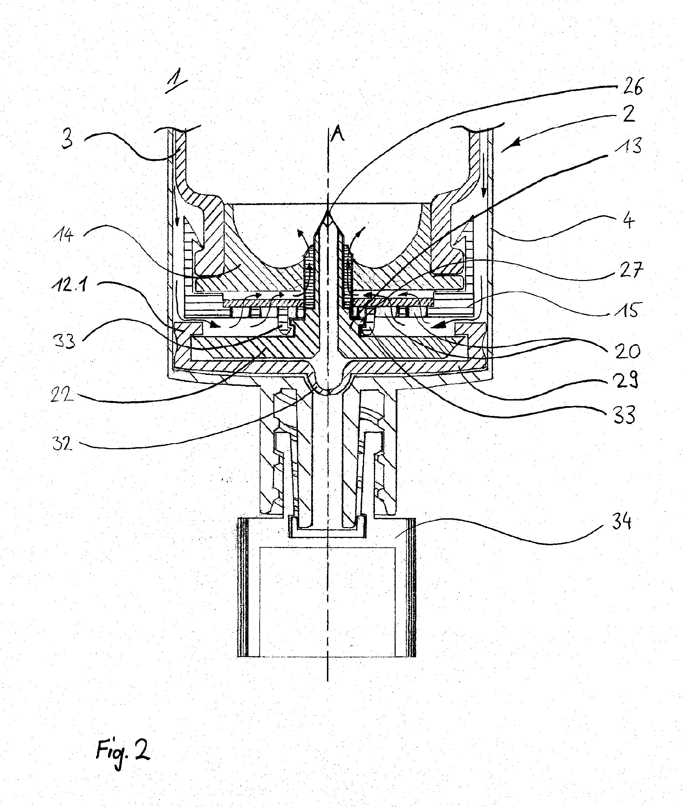

Um den Durchbruch 17 sind am Zentrierring 18 nach vorne ragende Rasthaken 33 ausgebildet, welche derart bemessen sind, dass sie den Kragen 27 des Penetrationselements 13 übergreifen können und somit als Verrastung des Kolbens 12 am Aufsatz 15 und damit am Behälter 3 dienen können (siehe hierzu Fig. 2).

Das vorzugsweise rotationssymmetrisch ausgebildete Penetrationselement 13 ist an eine senkrecht zur Längsrichtung A angeordnete Basisplatte 22 angeformt. Die Basisplatte 22 weist einen zentralen Durchbruch 23 auf, welcher in einen Fluidkanal 24 der Hohlnadel 25 übergeht. Nahe einer Wurzel der Hohlnadel 25 ist ein Kragen 27 ausgebildet, welcher von der Basisplatte 22 beabstandet ist. Das Penetrationselement 13 und die Basisplatte 22 sind dabei vorzugsweise einstückig ausgebildet. Mit Vorteil ist das Penetrationselement 13 als Hohlnadel 25 ausgestaltet und ragt in Richtung zum Behälter 3. Der Fluidkanal 24 mündet in diesem Fall an einer mit Öffnungen versehenen Aufstechspitze 26 der Hohlnadel 25.

Der Begriff Hohlnadel 23 wird hier allgemein für jedes Element verwendet, das einen Ansaugkanal 24 bildet und das in der Lage ist ein Verschlusselement 14 zu penetrieren. In der Lagerstellung der Fig. 1 ist die Aufstechspitze 26 im zentralen Durchbruch 27 des Aufsatzes 25 vor dem Verschlusselement 24 angeordnet.

Die Hohlnadel 25 hat eine relativ dicke Wandung, entlang welcher vorzugsweise parallele Belüftungskanäle 28 in der Form von Nuten angeordnet sind. Diese Nuten erstrecken sich bis beinahe an den Kragen 27 heran. Die Belüftungskanäle können im Querschnitt auch als Schwalbenschwanznut ausgebildet sein. Damit kann die Gefahr reduziert werden, dass das elastische Material des Verschlusselements 14 die Belüftungskanäle 28 verschliesst.

Die Basisplatte 22 ist in einer Kolbenplatte 29 des Kolbens 12 gehalten. Ein Querschnitt der Kolbenplatte 29 entspricht im Wesentlichen einem Querschnitt des Innenraums 11 und weist mantelseitig Dichtungsrippen auf, sodass die Kolbenplatte 29 den Innenraum 11 in zwei Abschnitte unterteilt. Ein vor der Kolbenplatte 29 angeordneter Abschnitt, d.h. zwischen Kolbenplatte 29 und Abschluss 6, bildet dabei ein Abgabevolumen 30 während ein hinter der Kolbenplatte 29 angeordneter Abschnitt ein Vorratsvolumen 31 zur Aufnahme des Behälters 3 bildet. In der Lagerstellung gemäss der Fig. 1 ist das Abgabevolumen 30 im Wesentlichen verschwindend, da die Kolbenplatte 29 in dieser Stellung ganz nach vorne bis an eine Innenseite des Abschlusses 6 heran verschoben ist.

Die Kolbenplatte 29 ist am Rand mit einem nach hinten ragenden, klauenartig ausgebildeten Kragen 12.1 versehen. Die Basisplatte 22 des Penetrationselements 13 ist in den Kragen 12.1 eingeschnappt, sodass die Basisplatte 22 mit Penetrationselement 13 und Kolbenplatte 29 eine starre Einheit bilden. Grundsätzlich ist es allerdings auch denkbar, dass Kolbenplatte 29 und Basisplatte 22 zusammen mit dem Penetrationselement 13 einstückig ausgebildet sind oder die Basisplatte 22 in die Kolbenplatte 29 eingeschweisst oder an diese angespritzt ist.

In einem zentralen Bereich direkt vor dem Durchbruch 23 der Basisplatte 22 ist die Kolbenplatte 29 ausgedünnt und als Kalotte 32 nach vorne gewölbt. Hierbei ist bei der Austrittsöffnung 7 ein entsprechender Aufnahmeraum ausgebildet, in welchen die Kalotte 32 bei ganz noch vorne verschobenem Kolben 12 aufgenommen ist. In einem Bereich am vordersten Punkt der Kalotte 32 ist ein Schlitz ausgebildet. Die Kalotte 32 bildet somit ein Domventil, welches einen Flüssigkeitsdurchtritt vom Fluidkanal 8 bzw. dem Durchtritt 23 nach vorne, zur Abgabeöffnung 7 hin erlaubt, aber in Gegenrichtung sperrt. Die Kalotte 32 ist hierzu elastisch ausgebildet, sodass der Schlitz von selbst schliesst.

In der Darstellung der Fig. 1 ist die Kalotte 32 einstückig mit der Kolbenplatte 29 ausgebildet. Die bereichsweise unterschiedlichen Elastizitätsanforderungen des Kolbens 12 können bei geeigneter Materialwahl durch gezielte Ausdünnung oder Verdickung der einzelnen Bereiche erreicht werden. Es versteht sich, dass die Kalotte 32 auch als separates Teil ausgebildet und in die Kolbenplatte 29 eingesetzt oder an diese angespritzt sein kann.

Figur 2 zeigt die Anordnung 1 gemäss Figur 1 in einer Entnahmelage. Gegenüber der Lagerstellung der Figur 1 ist der Behälter im Innenraum 11 des Hohlkörpers 4 nach vorne verschoben. Dies kann beispielsweise durch Druck von einem Anwender auf ein hinteres freies Ende des Behälters 3 bewirkt werden. Durch das nach vorne Schieben ist der Behälter 3 gegen den Kolben 12 verschoben, sodass die Anstechspitze 26 das Verschlusselement 14 durchstossen hat und in das Innere des Behälters 3 ragt. Das Penetrationselement 13 ist somit wenigstens teilweise im Verschlusselement 14 angeordnet. Die Belüftungskanäle 28 schaffen dabei eine Verbindung zwischen dem Innenraum des Behälters 3 und dem Belüftungskanal 19 des Aufsatzes 15.

In der Entnahmelage der Figur 2 umgreifen die Rasthaken 33 den Kragen 27 des Penetrationselements 13 und verankern so den Kolben 12 am Behälter 3. Der Kragen 27 dient dabei gleichzeitig als Anschlag für das nach vorne Schieben des Behälters 3, indem eine Stirnseite des Aufsatzes 15 am Kragen 27 anstösst. Rasthaken 33 und Kragen 27 sind derart ausgebildet, dass beim Anstossen des Aufsatzes 15 an den Kragen 27 die Rasthaken 33 mit diesem verrasten und Kolben 12 und Behälter 3 bezüglich einer Längsverschiebung im Innenraum 11 zwangsgekoppelt sind.

Die Basisplatte 22 sowie die Kolbenplatte 29 sind dabei derart ausgebildet, dass die Ansaugöffnungen 20 frei sind und mit dem Vorratsbereich 31 des Hohlkörpers 4 kommunizieren. Dies wird insbesondere dadurch sichergestellt, dass zum einen der Kragen 27 von der Basisplatte 22 derart beabstandet ist, dass die Stirnseite des Aufsatzes 15 nicht auf der Basisplatte 22 aufliegen kann. Entsprechend ist ein äusserer Rand der Kolbenplatte 29 derart ausgestaltet, dass zwischen Aufsatz 15 und Kolbenplatte 29 ein Luftspalt besteht. Luft aus der Atmosphäre kann somit am Behälter 3 vorbei durch den Vorratsbereich 31 zwischen den Behälter 3 und die Kolbenplatte 29 strömen, wo sie durch die Ansaugöffnungen 20 und durch das Filter 21 in den Belüftungskanal 19 eintritt und von dort durch die Belüftungskanäle 28 des Penetrationselements 13 in das Innere des Behälters 3 gelangen kann.

In beiden Figuren 1 und 2 ist die Abgabeöffnung 7 des Hohlkörpers 4 luftdicht durch eine auf das Luer-Lock 10 aufgesetzte Kappe 34 verschlossen.

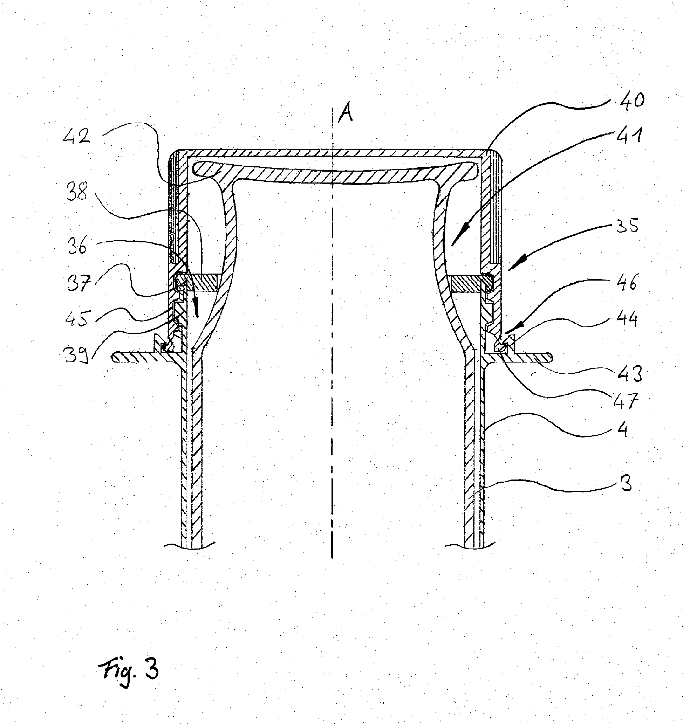

Figur 3 zeigt einen Ausschnitt im hinteren Bereich der Anordnung 1 in einer der Fig. 1 entsprechenden Ansicht. An einem hinteren Längsende 35 des Hohlkörpers 4 ist eine Zugangsöffnung 36 ausgebildet, welche über den gesamten Querschnitt des Innenraums 11 des Hohlkörpers 4 nach hinten offen ist. Durch die Zugangsöffnung 36 ragt ein hinteres Ende des Behälters 3 über den Hohlkörper 4 hinaus. Im hinteren Bereich weist der Behälter 3 eine Einschnürung 41 auf, welche sich am hinteren Längsende zu einer Druckplatte 42 für die Bedienung der Anordnung 1 aufweitet. Ein Querschnitt der Druckplatte 42 entspricht dabei weitgehend dem Querschnitt des Behälters 3, sodass der gesamte Behälter 3 innerhalb einer gedachten zylindrischen Fortsetzung seiner vorderen Mantelfläche angeordnet ist. Aufgrund der Verjüngung 41 dient die Druckplatte 42 gleichzeitig als Fingerflansch beim Herausziehen des Behälters 3 aus dem Hohlkörper 4.

An einem Rand 37 der Zugangsöffnung 36 ist ein nach innen auskragender elastischer Ring 38 angebracht. Der Ring 38 ist von hinten her auf den als Haltemittel ausgebildeten Rand 37 aufgesetzt, wobei der Ring 38 auch radial nach aussen geringfügig über den Hohlkörper 4 übersteht. Der Ring 38 weist dabei Durchbrüche auf (nicht dargestellt) wie z.B. eine Zahnung, sodass Luft in den Vorratsbereich 31 des Hohlkörpers 4 strömen kann, auch wenn der Ring 38 am Behälter 3 anliegt (z.B. wenn dieser zurückgezogen ist).

In der Lagerstellung ist der Behälter 3 derart im Hohlkörper 4 angeordnet, dass der Ring 38 im Bereich der Einschnürung 41 angeordnet ist, sodass der Behälter 3 vor einem Herausfallen aus der Vorrichtung 2 geschützt ist. Es versteht sich, das Behälter 3 und Vorrichtung 2 derart ausgebildet und aneinander angepasst sind, dass in dieser Lage das Penetrationselement 13 in der Stellung der Figur 1 knapp vor dem Verschlusselement 14 angeordnet ist.

Aussenseitig, vor dem Ring 38 weist der Hohlkörper 4 einen nach aussen auskragenden Fingerflansch 43 auf, welcher als Ablage für die Finger eines Anwenders zur Erzeugung einer Gegenkraft beim Druck auf die Druckplatte 42 des Behälter 3 dient. In Längsrichtung zwischen Fingerflansch 43 und Ring 38 ist ein aussen umlaufender Rastvorsprung 39 ausgebildet, welcher als Verrastung für eine Verschlusskappe 40 dient. Der Fingerflansch 43 weist zudem auf einer hinteren Seite einen umlaufenden Rastkragen 44 auf, welcher von einer Mantelfläche des Hohlkörpers 4 radial etwas beabstandet ist und als Gegenverrastung für einen Rastflansch 47 der Verschlusskappe 40 dient.

Die Verschlusskappe 40 ist von hinten her über die Zugangsöffnung 36 gestülpt und überspannt den Behälter 3, sodass dieser vor einer unbeabsichtigten Betätigung geschützt ist. Die Verschlusskappe 40 greift mit dem radial nach aussen auskragenden Rastflansch 47 in den Rastkragen 44 ein und ist somit gegen ein Abziehen gesichert. Eine innen umlaufende Rastnut 45 der Verschlusskappe 40 ist mit dem Rastvorsprung 39 im Eingriff. Der nach aussen überstehende Teil des Rings 38 liegt innenseitig an der Verschlusskappe 40 an und erfüllt somit neben seiner Haltefunktion für den Behälter 3 auch eine Dichtfunktion zur sterilen Versiegelung der Anordnung 1.

In Längsrichtung zwischen Rastkragen 44 und Rastnut 45 ist eine umlaufende Sollbruchstelle 46 an der Verschlusskappe 40 ausgebildet. Vor Gebrauch der Anordnung wird die Verschlusskappe 40 längs der Sollbruchstelle 46 abgerissen, wobei der Rastkragen 44 als Garantieband am Hohlkörper 4 verbleibt. Es versteht sich, dass die Verschlusskappe 40 auch auf andere Weise am Fingerflansch befestigt sein kann wie z.B. angeschweisst oder verklebt. Die Verschlusskappe 40 kann aber zur Benutzung auch gesamthaft abgenommen werden.

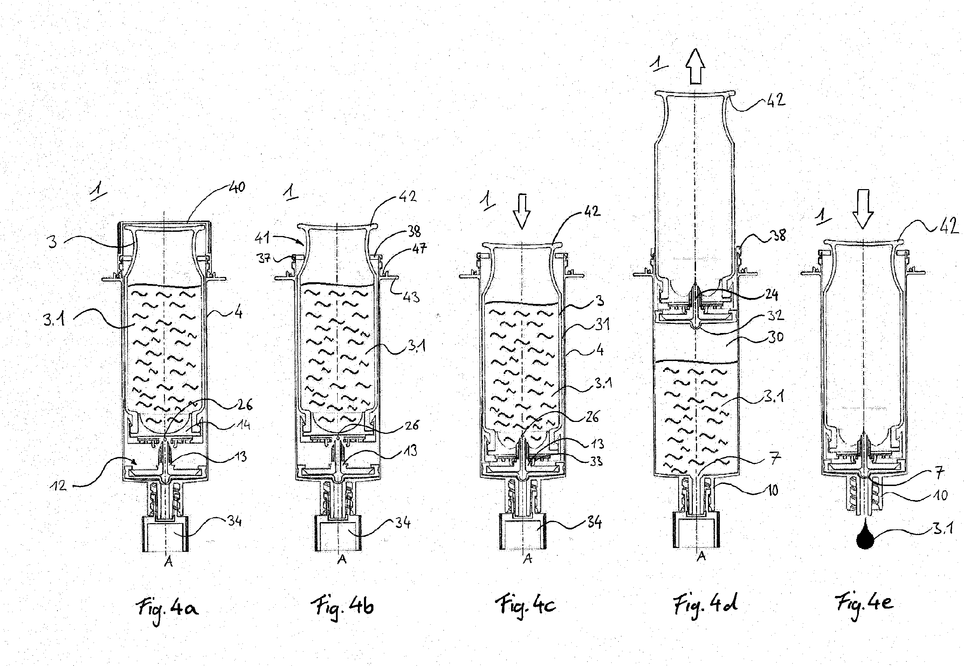

Figuren 4a bis 4e zeigen den Ablauf bei der Benutzung der Erfindungsgemässen Anordnung 1.

Figur 4a zeigt die Anordnung 1 in der Lagerstellung mit daran vorhandener Verschlusskappe 40 gemäss den Figuren 1 und 3. Die Abgabeöffnung ist mit der Kappe 34 verschlossen.

Vor Gebrauch wird die Verschlusskappe 40 längs der Sollbruchstelle 46 abgenommen. Figur 4b zeigt die Anordnung 1 ohne die Verschlusskappe 40. Der Rastkragen 47 verbleibt dabei als Garantieband am Fingerflansch 43. Der Behälter 3 ragt mit seinem hinteren Längsende durch die Zugangsöffnung 36 über den Hohlkörper 4 hinaus, sodass die Druckplatte 42 für einen Anwender zugänglich ist.

Durch Druck auf die Druckplatte 42 nach vorne wird der Behälter 3 von einem Anwender im Hohlkörper 4 nach vorne gegen den Kolben 12 verschoben. Der Fingerflansch 43 dient dabei als Fingerrast zur Ausübung einer Gegenkraft, während der Druck auf die Druckplatte 42 typischerweise mit einem Daumen ausgeübt wird. Die Aufstechspitze 26 durchdringt dabei das Verschlusselement 14 und die Rasthaken 33 verrasten mit dem Kragen 27. Stösst der Behälter an den Kragen 27 und ist der Kolben 12 in dieser Stellung mit dem Behälter 3 verrastet, so ist die Anordnung 1 in der Entnahmelage wie in Figur 4c gezeigt (entspricht der Stellung der Anordnung 1 der Figur 2).

Wird der Behälter 3 an der Druckplatte 42 nach hinten, d.h. aus dem Hohlkörper 4 heraus, gezogen, entsteht im Abgabebereich 30 aufgrund der Bewegung des Kolbens 12 und der verschlossenen Abgabeöffnung 7 ein Unterdruck. Aufgrund des Unterdrucks strömt die Flüssigkeit 3.1 aus dem Behälter 3 durch den Fluidkanal 24 sowie durch das von der Kalotte 32 gebildete Domventil in den Abgabebereich 30. Auf dem im Zusammenhang mit Figur 2 beschriebenen Weg fliesst aufgrund des so entstehenden Unterdrucks im Behälter 3 Luft aus der Atmosphäre durch den Vorratsbereich 31 in den Behälter 3. Bei vollständig zurückgezogenem Behälter 3 ist die gesamte Flüssigkeit aus dem Behälter 3 in den Abgabebereich überführt worden. Die Anordnung 1 ist damit in einem abgabebereiten Zustand bzw. in einer Abgabestellung. Es versteht sich, dass die Anordnung in der Abgabestellung sein kann, wenn auch nur ein Teil der Flüssigkeit im Abgabebereich vorhanden ist.

Es versteht sich, dass der Hohlkörper 4 z.B. transparent ausgebildet sein kann, sodass ein Flüssigkeitsstand im Abgabebereich 30 überprüft werden kann. Aussenseitig am Hohlkörper 4 kann hierzu eine Skala aufgedruckt sein.

In Figur 4e ist die Kappe 34 vom Luer-Lock 10 entfernt, womit die Abgabeöffnung 7 offen ist. Durch Druck auf die Druckplatte 42 nach vorne wird der Kolben 12 wieder nach vorne geschoben. Die als Domventil wirkenden Kalotte 32 sperrt gegen einen Fluss der Flüssigkeit 3.1 zurück in den Fluidkanal 24 bzw. den Behälter 3. Der Kolben 12 wirkt daher wie der Kolben einer herkömmlichen Spritze und presst die Flüssigkeit 3.1 durch die Abgabeöffnung 7 nach aussen. Nach dem Gebrauch kann die Anordnung 1 entsorgt werden.

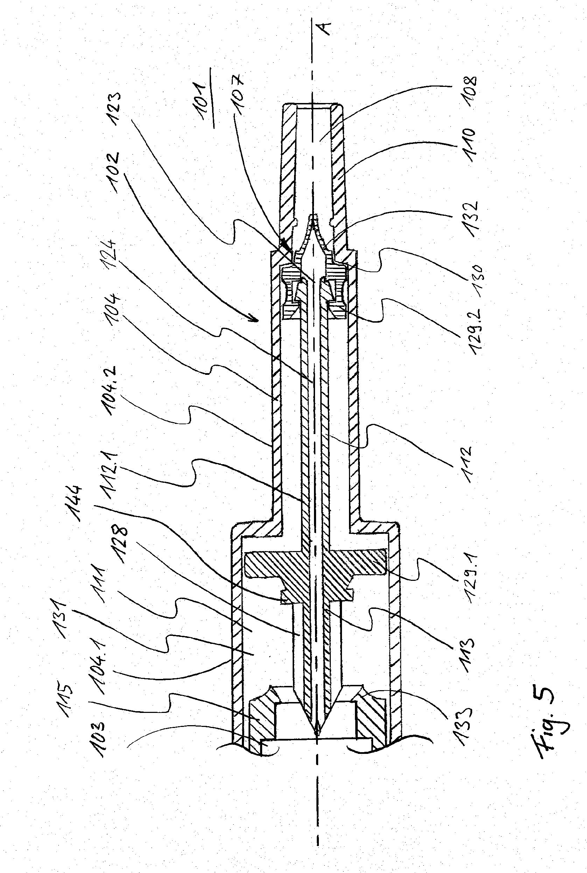

Figur 5 zeigt eine weitere Ausführungsform einer erfindungsgemässen Anordnung 101 mit einer Vorrichtung 102 sowie einem Behälter 103.

Ein Hohlkörper 104 ist in diesem Fall abschnittweise zylindrisch ausgebildet, mit einem ersten, hinteren Abschnitt 104.1 grösseren Querschnitts und einem zweiten, vorderen Abschnitt 104.2. Ein Vorratsbereich 131 in einem Innenraum 111 des Hohlkörpers 104 ist dabei im hinteren Abschnitt 104.1 angeordnet, während ein Abgabebereich 130 im vorderen Abschnitt 104.2 angeordnet ist.

Ein Kolben 112 der Vorrichtung 102 erstreckt sich dabei vom hinteren 104.1 in den vorderen Abschnitt 104.2. Hierzu weist der Kolben 112 eine erste Kolbenplatte 129.1 grösseren Querschnitts auf, welche im hinteren Abschnitt 104.1 in Längsrichtung A der Vorrichtung verschiebbar geführt gelagert ist. Eine zweite Kolbenplatte 129.2 des Kolbens 112 ist im vorderen Abschnitt 104.2 mantelseitig fluiddicht in Längsrichtung A verschiebbar geführt gelagert. Die beiden Kolbenplatten 129.1 und 129.2 sind über eine Kolbenstange 112.1 starr miteinander verbunden, wobei ein Fluidkanal 124 des Kolbens 112 sich in der Kolbenstange 112.1 von der hinteren 129.1 zur vorderen Kolbenplatte 129.2 erstreckt. Der Fluidkanal 124 ist dabei an einem Durchbruch 123 der vorderen Kolbenplatte 129.2 offen. Die vordere Kolbenplatte 129.2 weist ein Schnabelventil 132 auf, welches den Durchbruch 123 verschliesst und gegen einen Durchtritt eines Fluidums aus dem Abgabebereich in den Fluidkanal sperrt. In der Darstellung der Figur 5 ist die vordere Kolbenplatte 129.2 einstückig mit dem Schnabelventil 132 ausgebildet, wobei es sich versteht, dass in anderen Ausführungsformen die Kolbenplatte 129.2 und Ventil 132 auch mehrteilig ausgebildet sein können. Ebenso kann die gemäss Figur 5 auf die Kolbenstange aufgeschnappte Kolbenplatte 129.2 in anderen Ausführungsformen auch einstückig mit der Kolbenplatte 129.2 ausgebildet sein. Anstelle des Schnabelventils sind auch Domventile oder andere analoge Ventile denkbar.

Der vordere Bereich 104.2 weist an einem vorderen Längsende des Hohlkörpers 104 eine Abgabeöffnung 107 auf, welcher ein Aussenkonus einer Luer-Kupplung 110 mit einem Fluidkanal 108 zur Abgabe einer Flüssigkeit aus dem Abgabebereich 130 vorgelagert ist. Vorteilhaft kann eine Nadel auch direkt in die Abgabeöffnung eingesetzt sein.

Die hintere Kolbenplatte 129.1 weist ein nach hinten ragendes Penetrationselement 113 in Form einer Hohlnadel 125 auf, welche einem Kopplungsbereich eines Aufsatzes 115 des Behälters 103 zugewendet ist. Der Behälter 3 ist an einer Behältermündung von einem Verschlusselement verschlossen, welches zur Penetration durch die Hohlnadel 125 vorgesehen ist (nicht dargestellt). Die Hohlnadel 115 weist analog dem Penetrationselement 13 der vorherig beschriebenen Ausführungsform Belüftungskanäle 128 auf. Der Behälter 103 weist ebenfalls einen (nicht dargestellten) Belüftungskanal am Aufsatz 115 auf, bevorzugt mit einem Filter. Insbesondere kann der Behälter 103 mit Aufsatz 115 gesamthaft entsprechend dem Behälter 3 ausgebildet sein.

Der Kolben 112 weist analog dem zuvor beschriebenen Kolben 12 der Figuren 1-4 an der Wurzel des Penetrationselements 113 einen Rastkragen 144 auf, welcher als Kopplungsmittel zum Ankoppeln an entsprechende Kopplungsmittel 133 des Behälters 103 dient.

Die Anwendung der Anordnung 101 geschieht analog zu dem in Figuren 4a-4e beschriebenen Abläufen. Durch die abschnittweise unterschiedliche Ausbildung des Hohlkörpers 104, insbesondere eines Innenraums des Hohlkörpers 104 wird erreicht, dass ein Innenquerschnitt des Abgabebereichs 130 von eine Dimensionierung des Penetrationselements 113 und der in diesem Bereich ausgebildeten Kopplungsvorrichtungen des Kolbens 112 sowie des Behälters 103 unabhängig ist. Insbesondere kann im hinteren Abschnitt 104.1 ein grösserer Querschnitt gewählt werden, um Behälter 103 sowie die Kopplungsmechanik einfach im hinteren Abschnitt 104.1 unterbringen zu können. Im vorderen Abschnitt 104.2 kann hingegen ein kleinerer Querschnitt gewählt werden, sodass auch eine geringe zur Abgabe vorgesehen Flüssigkeitsmenge genau und fein dosiert werden kann. Der vordere Bereich 104.2 kann hierzu transparent ausgebildet sein und im Abgabebereich 130 zudem eine Feindosierungs-Skala umfassen.

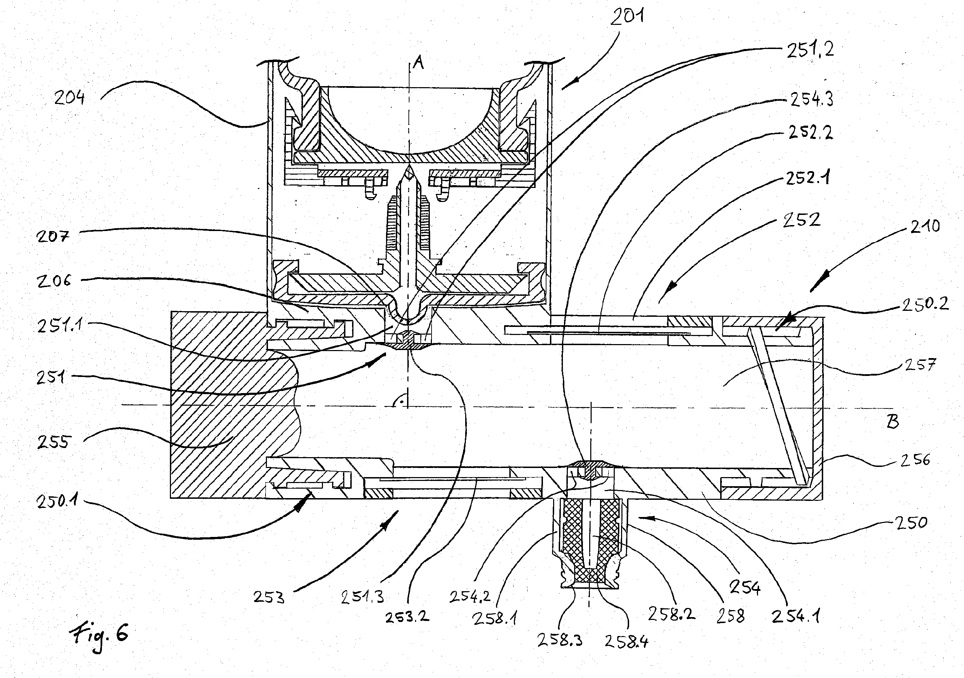

Figur 6 zeigt eine weitere Ausführungsform einer erfindungsgemässen Anordnung 201 mit einem Anschlussstück 210 zum Anschluss an ein fluidführendes System.

Das Anschlussstück 210 ist als länglicher Rohrabschnitt 250 ausgebildet und direkt an die Anordnung 201 angeformt. Eine Längsrichtung B des Rohrabschnitts 250 ist weitgehend senkrecht zur Längsachse A der Anordnung 201 angeordnet direkt an einen stirnseitigen Abschluss 206 eines Hohlkörpers 204 der Anordnung 201 angeformt. Die Anordnung 201 entspricht dabei weitgehend der Ausführungsform der Figuren 1 bis 4, weshalb sie hier nicht erneut beschrieben ist.

An den Endseiten ist der Rohrabschnitt 250 mit jeweils einer Kopplungsstelle 250.1 und 250.2 versehen, welch einen Anschluss des Rohrabschnitts 250 an das fluidführende System (nicht dargestellt) erlaubt. In der Darstellung der Figur 6 ist die Kopplungsstelle 250.1 als Schnappkupplung ausgebildet und mit einer entsprechenden abnehmbaren Verschlusskappe 255 versehen. Die Kopplungsstelle 250.2 ist als Gewindekupplung ausgebildet und weist ebenfalls eine abnehmbare Verschlusskappe 256 auf.

Eine Abgabeöffnung 207 der Anordnung 201 mündet direkt in einen Fluidkanal 251.1 einer Zugangsöffnung 251 des Rohrabschnitts 250. Der Fluidkanal 251.1 bildet dabei einen Durchtritt durch eine Rohrwand des Rohrabschnitts 250 in einer Richtung senkrecht zur Längsachse B, d.h. weitgehend parallel zu A. Innenseitig an der Rohrwand weist die Zugangsöffnung 251 mehrere Öffnungen 251.2 auf, welche einen Durchtritt von Flüssigkeit aus dem Fluidkanal 251.1 in einen Innenraum 257 des Rohrabschnitts 250 ermöglichen. Die Öffnungen 251.2 sind dabei bevorzugt ringförmig um eine zentral angeordnete Befestigungsstelle eines Schirmventils 251.3 angeordnet. Ein Schirm des Schirmventils 251.3 erstreckt sich in den Innenraum 257 und ist derart ausgebildet, dass er die Öffnungen 251.2 vollständig überdeckt. Der Schirm verjüngt sich radial von der Befestigungsstellt ausgehend nach aussen, sodass an einem Rand ein weitgehend glatter Übergang zur Innenwand des Rohrabschnitts 250 besteht.

Wird nun die Anordnung 201 betätigt, gelangt die zur Abgabe vorgesehene Flüssigkeit in den Fluidkanal 251.1. Ist ein Druck in der Flüssigkeit genügend gross, wird der Schirm des Schirmventils 251.3 von der Rohrinnenwand abgehoben, sodass die Öffnungen 251.2 freigegeben werden und die Flüssigkeit in den Innenraum 257 gelangt. Sobald kein Druck mehr in der Flüssigkeit vorhanden ist, schliesst das Schirmventil 251.3, indem der Schirm sich wieder dichtend über die Öffnungen 251.2 legt.

Der Schirm ist bevorzugt als dünne Dichtlippe ausgebildet, welche sich innenseitig an die Rohrwand schmiegt. Durch den weitgehend glatten Übergang von der Rohrwand auf den Schirm ergibt sich gesamthaft eine nur geringfügige Beeinträchtigung einer im Innenraum 257 des Rohrabschnitts 250 strömenden Flüssigkeit des fluidführenden Systems. Ebenso ist unmittelbar ersichtlich, dass im Innenraum 257 aufgrund der erfindungsgemässen Konstruktion keine Toträume vorhanden sind, in welchen die strömende Flüssigkeit auf unerwünschte Weise verweilen könnte.

Das Anschlussstück 210 weist zudem drei Injektionsports 252 bis 254 auf. Ein direkt neben der Anordnung 201 ausgebildeter Injektionsport 252 umfasst ein in eine Öffnung 252.1 der Rohrwand eingesetztes Septum 252.2. Das Septum 252.2 erlaubt eine selbstschliessende Penetration durch beispielsweise eine Injektionsnadel.

Das Septum 252.2 ist derart in der Rohrwand ausgebildet, dass es eine weitgehend direkte Fortsetzung der Rohrwand bildet und so eine strömende Flüssigkeit möglichst nicht behindert bzw. verwirbelt. Das Septum 252.2 ist hierzu direkt an der Rohrwand befestigt wie beispielsweise ultraschall- oder laser-verschweisst.

Bezüglich der Längsachse B der Zugangsöffnung 251 gegenüberliegend ist in der Rohrwandung ein weiterer Injektionsport 253 ausgebildet. Der Injektionsport 253 ist weitgehend analog zum Injektionsport 252 ausgebildet und umfasst ein Septum 253.2.

Bezüglich der Längsachse B dem Injektionsport 252 gegenüberliegend ist in der Rohrwandung ein weiterer Injektionsport 254 ausgebildet. Dieser weist analog der Zugangsöffnung 251 einen Fluidkanal 254.1 mit Öffnungen 254.2 sowie einem Schirmventil 254.3 auf. Im Gegensatz zur Zugangsöffnung 251 ist aussenseitig am Rohrabschnitt 250 allerdings ein Kopplungsmittel 258 vorgesehen, welches eine nadellose Ankopplung einer medizinischen Einrichtung zur Flüssigkeitsabgabe ermöglicht. In der Darstellung der Figur 6 ist das Kopplungsmittel 258 als so genanntes abtupfbares Ventil ausgebildet ("swabable valve"). Derartige Ventile umfassen einen Kupplungssockel 258.1, welcher vorliegend am Rohrabschnitt angeformt ist. Ein mit dem Fluidkanal 253.1 in Kommunikation stehender Innenraum des Sockels 258.2 weist eine Anschlussöffnung 258.3 mit z.B. einem Innenkonus einer Luer-Kupplung auf. Zur Dichtung gegen Verschmutzung oder Kontamination ist im Innenraum ein Schlitzventil 258.4 angeordnet, welches bei Einbringen eines Aussenkonus zurückgedrängt wird und bei Ausbringen die äussere Öffnung 258.3 wieder verschliesst. Das Schlitzventil 258.4 ist derart in der Öffnung 258.3 angeordnet, dass es von aussen auf einfache Weise abgetupft d.h. gereinigt werden kann. Derartige Kopplungen sind in der US 6,651,956 (Halkey-Roberts Corporation) beschrieben.

Es versteht sich, dass der Rohrabschnitt 250 beispielsweise auch in ein Rohr oder einen Schlauch integriert sein kann. Ebenso kann der Abschnitt z.B. einseitig fest verschlossen sein und nur an einem Ende zur Ankopplung an ein fluidführendes System vorgesehen sein. Ebenso ist unmittelbar ersichtlich, dass auch andere Kopplungsmittel wie z.B. Luer-Lock und auch zusätzliche Injektionsports vorgesehen sein können. Im Allgemeinen empfiehlt es sich, dass der Rohrabschnitt zusätzlich zur erfindungsgemässen Vorrichtung wenigstens einen weiteren Port aufweist, an welche eine weitere Abgabevorrichtung angekoppelt werden kann.

Aus der Figur 6 wird unmittelbar klar, dass das Anschlussstück auch als ein separates Teil ausgebildet sein kann, d.h. nicht direkt an eine Vorrichtung angeformt sein muss. Die Ausbildung mit einem Schirmventil zum Verschliessen der Zugangsöffnung ist auch in diesem Fall vorteilhaft und verhindert weitgehend eine unerwünschte Verwirbelung der strömenden Flüssigkeit sowie allfällige Toträume im Innenraum des Rohrabschnitts. In diesem Fall ist die Zugangsöffnung 251 ebenfalls als ein Injektionsport beispielsweise gemäss dem Injektionsport 254 ausgebildet. Dem Fachmann ergeben sich unmittelbar aus Figur 6 mögliche weitere Ausführungsformen eines separaten Anschlussstücks.

## Patentansprüche

1. Vorrichtung (2) für die Abgabe einer Flüssigkeit aus einem Behälter (3), welcher mit einem penetrierbaren Verschlusselement (14) verschlossen ist,
a.) umfassend einen rohrförmigen Hohlkörper (4, 204) mit einem Innenraum (11), welcher an einem Längsende einen Abschluss mit einer Abgabeöffnung (7, 207) aufweist,
b.) und einem im Innenraum (11), mantelseitig fluiddicht, längsverschiebbar angeordneten Kolben (12), welcher im Innenraum (11) einen Vorratsbereich (31) und einen Abgabebereich (30) abgrenzt,
c.) wobei der Vorratsbereich (31) nach aussen offen ist und mit der Atmosphäre kommuniziert, und zur ganz oder teilweisen längsverschiebbaren Aufnahme des Behälters (3) ausgebildet ist,
d.) und der Abgabebereich (30) für eine Aufnahme einer zur Abgabe vorgesehenen Flüssigkeitsmenge vorgesehen ist und an die Abgabeöffnung (7, 207) anschliesst,
e.) wobei der Kolben (12) ein Kopplungsmittel (27) umfasst, mit welchem der Kolben (12) an ein entsprechendes Kopplungsmittel (33) des Behälters (3) bezüglich einer Längsverschiebung koppelbar ist,
f.) und weiter ein Penetrationselement (13) aufweist, mit welchem das Verschlusselement (14) penetrierbar ist, wenn der Kolben (12) an den Behälter (3) gekoppelt ist,
g.) wobei das Penetrationselement (13) einen Fluidkanal (24) aufweist, welcher einen Flüssigkeitsdurchtritt in den Abgabebereich (30) ermöglicht,
**dadurch gekennzeichnet, dass**
h.) das Penetrationselement (13) einen Belüftungskanal (28) zum Belüften des Behälters (3) bei Entnahme der Flüssigkeit umfasst, welcher mit dem Vorratsbereich (31) kommuniziert und durch welchen dem Behälter (3) über den Vorratsbereich (31) Luft aus der Atmosphäre zuführbar ist.

2. Vorrichtung nach Anspruch 1, **dadurch gekennzeichnet, dass** sie wenigstens ein Filter umfasst, über das Luft aus der Atmosphäre über den Belüftungskanal (28) in den Behälter (3) zuführbar ist.

3. Vorrichtung nach Anspruch 1 oder 2, **dadurch gekennzeichnet, dass** der Belüftungskanal (28) als Nut auf einer Aussenseite des Penetrationselements (13) ausgebildet ist und insbesondere das Penetrationselement (13) eine Hohlnadel (25) umfasst, deren Hohlraum zumindest einen Teil des Fluidkanals (24) bildet.

4. Vorrichtung nach einem der Ansprüche 1 bis 3, **dadurch gekennzeichnet, dass** ein Ventil (32) ausgebildet ist, insbesondere eine Domventil (32), welches einen Durchtritt von Flüssigkeit durch den Fluidkanal (24) in den Abgabebereich (30) zulässt und in Gegenrichtung sperrt.

5. Vorrichtung nach einem der Ansprüche 1 bis 4, **dadurch gekennzeichnet, dass** das Kopplungsmittel (27) des Kolbens (12) als Teil einer Schnapp-Kupplung zum verrastenden Eingriff mit dem entsprechenden Kopplungsmittel (33) des Behälters (3) ausgebildet ist.

6. Vorrichtung nach einem der Ansprüche 1 bis 5, **dadurch gekennzeichnet, dass** der Hohlkörper (4, 204) an einem dem Abschluss (6) gegenüber liegenden Längsende eine Zugangsöffnung (36) aufweist, welche mit der Atmosphäre kommuniziert und einen Zugang zum Vorratsbereich (31) bildet und derart ausgestaltet ist, dass der Behälter (3) durch die Zugangsöffnung (36) in den Vorratsbereich (31) einbringbar und/oder zum Verschieben im Innenraum (11) von aussen manipulierbar ist.

7. Vorrichtung nach einem der Ansprüche 1 bis 6, **dadurch gekennzeichnet, dass** der Hohlkörper (4, 204) aussenseitig an der Abgabeöffnung (7, 207) ein Kupplungselement (10, 210) aufweist.

8. Vorrichtung nach Anspruch 7, **dadurch gekennzeichnet, dass** das Kupplungselement (210) einen weitgehend quer zur Längsachse des Hohlkörpers (204) angeordneten Rohrabschnitt (250) mit einem zur Fluidführung vorgesehenen Lumen (257) umfasst, wobei die Abgabeöffnung (207) derart mit dem Lumen (257) kommuniziert, dass eine im Abgabebereich (30) vorhandene Flüssigkeit durch die Abgabeöffnung (207) in das Lumen (257) eingebracht werden kann.

9. Vorrichtung nach Anspruch 8, **dadurch gekennzeichnet, dass** der Rohrabschnitt (250) einstückig an den Hohlkörper (204) angeformt, insbesondere angespritzt, ist.

10. Vorrichtung nach Anspruch 8 oder 9, **dadurch gekennzeichnet, dass** der Rohrabschnitt (250) wenigstens an einem seiner Längsenden ein Kopplungselement (250.1, 250.2) zum Anschluss an eine fluidführende Komponente aufweist.

11. Vorrichtung nach einem der Ansprüche 8 bis 10, **dadurch gekennzeichnet, dass** der Rohrabschnitt (250) integraler Bestandteil eines zur Fluidführung vorgesehenen Systems, insbesondere eines Schlauchs, ist.

12. Vorrichtung nach einem der Ansprüche 8 bis 11, **dadurch gekennzeichnet, dass** der Rohrabschnitt (250) in einer Rohrwand wenigstens einen Injektionsport (252, 253, 254) mit einer Zugangsöffnung (251) zum Einbringen einer Flüssigkeit in das Lumen (257) umfasst, welcher bevorzugt einen Innenkonus eines Luer-Locks aufweist und insbesondere eine Ventilvorrichtung (251.3) vorhanden ist, vorzugsweise ein Schirmventil (251.3), welche einen Durchtritt von Flüssigkeit durch die Zugangsöffnung (251) in das Lumen (257) zulässt und in Gegenrichtung sperrt.

13. Behälter (3) mit einer Flüssigkeit (3.1) für eine Vorrichtung (2) gemäss einem der vorigen Ansprüche, welcher eine mit einem penetrierbaren Verschlusselement (14) verschlossenen Behältermündung (3.2) aufweist und am Verschlusselement (14) einen Aufsatz (15) mit einem Kopplungsmittel (27) zum Ankoppeln an den Kolben (12) umfasst, wobei der Aufsatz (15) in einem für die Penetration vorgesehenen Bereich des Verschlusselements (14) einen Durchtritt (17) zum Durchtritt des Penetrationselements (13) aufweist,
**dadurch gekennzeichnet, dass**
der Aufsatz (15) einen Belüftungskanal (19) aufweist, welcher am Durchtritt (17) offen ist, sodass einem im Durchtritt angeordneten Penetrationselement (13) durch den Belüftungskanal (19) Luft aus der Atmosphäre zuführbar ist.

14. Behälter nach Anspruch 13, **dadurch gekennzeichnet, dass** der Belüftungskanal (19) an einer Stirnseite des Aufsatzes (15) zur Atmosphäre hin offen ist.

15. Behälter nach Anspruch 13 oder 14, **dadurch gekennzeichnet, dass** der Aufsatz (15) ein Filter (21) umfasst, durch welches Luft aus der Atmosphäre über den Belüftungskanal (19) zu dem Penetrationselement (13), welches im Durchtritt (17) angeordnet ist, zuführbar ist.

16. Behälter nach Anspruch 15, **dadurch gekennzeichnet, dass** das Filter (21) in einer Halterung am Aufsatz (15) gehalten ist, welche den Durchtritt (17) des Aufsatzes (15) kreisringförmig umgibt.

17. Anordnung (1) mit einer Vorrichtung (2) nach einem der Ansprüche 1 bis 12 sowie mit einem eine Flüssigkeit enthaltenden Behälter (3), welcher eine mit einem penetrierbaren Verschlusselement verschlossene Behältermündung (3.2) aufweist und am Verschlusselement (14) einen Aufsatz (15) mit einem Kopplungsmittel (27) zum Ankoppeln an einen Kolben (12) der Vorrichtung (2) umfasst, insbesondere mit einem Behälter (3) gemäss einem der Ansprüche 13 bis 16, wobei der Behälter (3) wenigstens teilweise im Vorratsbereich (31) angeordnet ist.

18. Anordnung gemäss Anspruch 17, **dadurch gekennzeichnet, dass** die Anordnung (1) in eine Aufbewahrungsstellung und eine Entnahmelage bringbar ist, wobei der Behälter (3) in der Aufbewahrungsstellung derart im Vorratsbereich (31) angeordnet ist, dass das Penetrationselement (13) vom Verschlusselement (14) beabstandet ist und in der Entnahmelage der Aufsatz (15) des Behälters (3) an den Kolben (12) gekoppelt ist, wobei das Penetrationselement (13) das Verschlusselement (14) des Behälters (3) penetriert, wobei mit Vorteil in der Entnahmelage der Belüftungskanal (28) des Penetrationselements (13) derart bezüglich dem Belüftungskanal (19) des Aufsatzes (15) angeordnet ist, insbesondere derart im Durchtritt (17) des Aufsatzes (15) angeordnet ist, dass die Belüftungskanäle (19, 28) miteinander kommunizieren, sodass ein weitgehend durchgehender Gesamt-Belüftungskanal gebildet ist, durch welchen dem Behälter (3) über den Vorratsbereich (31) Luft aus der Atmosphäre zuführbar ist.

19. Anordnung gemäss Anspruch 18, **dadurch gekennzeichnet, dass** in der Entnahmelage die Belüftungskanäle (19, 28) derart zueinander angeordnet sind, dass die Luft aus der Atmosphäre bei der Zuführung zum Behälter (3) von dem Vorratsbereich (31) der Vorrichtung (2) durch den Belüftungskanal (19) des Aufsatzes (15) in den Belüftungskanal (28) des Penetrationselements (13) eintritt.

20. Anordnung gemäss einem der Ansprüche 17 bis 19 mit einem Behälter (3) mit den Merkmalen des Anspruchs 15, **dadurch gekennzeichnet, dass** das Filter (21) derart bezüglich der Belüftungskanäle (19, 28) angeordnet ist, dass die Luft aus der Atmosphäre zunächst durch das Filter (21) tritt, bevor sie in den Belüftungskanal (19, 28) des Aufsatzes und/oder des Penetrationselements (13) eintritt.
